Europäisches Patentamt

European Patent Office

Office européen des brevets

(11) Publication number: **0 173 181**
A1

## (12) EUROPEAN PATENT APPLICATION

(21) Application number: 85110262.4

(22) Date of filing: 16.08.85

(51) Int. Cl.⁴: **C 09 K 15/34, A 23 L 3/34**

(30) Priority: 16.08.84 JP 171214/84
11.09.84 JP 189967/84
12.10.84 JP 214670/84
04.10.84 JP 208545/84
26.12.84 JP 277839/84
20.06.85 JP 135781/85

(43) Date of publication of application: 05.03.86
Bulletin 86/10

(84) Designated Contracting States: CH DE FR GB IT LI

(71) Applicant: Fujikawa, Akio, 4-banchi, Fukakusa
Hiratamachi Fushimi-ku, Kyoto-City Kyoto (JP)
Applicant: Horiuchi, Tshio, 14-9, Kishibe
minami 3-chome, Suita-City Osaka (JP)

(72) Inventor: Fujikawa, Akio, 4-banchi, Fukakusa
Hiratamachi Fushimi-ku, Kyoto-City Kyoto (JP)
Inventor: Horiuchi, Tshio, 14-9, Kishibe minami 3-chome,
Suita-City Osaka (JP)

(74) Representative: Eitle, Werner, Dipl.-Ing. et al, Hoffmann,
Eitle & Partner Patentanwälte Arabellastrasse 4,
D-8000 München 81 (DE)

(54) Anti oxidation composition.

(57) This invention relates to Anti-oxidation composition consisting of the over ground parts of a carrot (Daucus carota L.) is useful applied for anti-oxidation agents, human cell activation agents, foods for care and growing hair, tonics for care and growing hair, composition of curing liver spots, healthy foods for eyes, foods for curing cataract, ingredient of tobacco composition and so on.

An anti-oxidation agents using for ingredient of foods or toilet goods in all of these usage have an especial advantage over a usual synthetic anti-oxidation agents such as α-tocopherol, BHA, BHT in storage characteristics.

Furthermore foods consisting of this Anti-Oxidation Composition have effects on the depression of the peroxide-lipids production in human body and high level safety for human beings.

Anti Oxidation Composition

Summary of the Invention

This invention relates to anti-oxidation composition consisting of the over ground parts of carrot (Daucus carota L.) is usefull applied for the various usage and has a good effect in each usages.

The usages of this anti-oxidation composition are anti-oxidation agents, human cell activation agents , foods for care and growing hair , tonics for care and growing hair , composition of curing liver spots , healty foods for eyes , foods for curing cataract , ingredient of tobacco composition , and so on.

Objects of the Invention

The object of this invention is to propose a very safety anti-oxidation composition made of an usual eatable vegitable , i.e. ground parts of carrot (Daucus carota L.).

More object of this invention is to propose an anti-oxidation composition having a wide application , i.e. anti-oxidation agents , human cell activation agents , foods for care and growing hair ; tonics for care and growing hair , composition of curing liver spots , healty foods for eyes , foods for cataract , ingredient of tobacco composition and so on.,.

Prior arts and defects of Prior arts

At present , synthtic or natural anti-oxidation agents are invariably added in foods or toilet goods in order to keep it a good characteristics.

Synthtic Chemicals, $\alpha$-tochopherol, B.H.A and B.H.T, as use as usual these anti-oxidation ingredients are said to give a bad-effect for human body.

With this problem in synthetic agents, vitamin E, as natural substance, comes into popular use as anti-oxidation agents for foods additives or the like at present.

Whereas Vitamin E has a superior anti-oxidation property, Continuous eating a large quantity of vitamin E takes human beings disadvantages on the ground of its oil-solubilty and accumulation in the body.

Generally, a carrot(Daucus carota L.) belonging the Umbelliferae family is one or two-years plants and native products area is said to be in the Middle East.

This carrot calutivated in china from the remotest days have been eating in Japan also for long time.

It goes with saying that contiuous eating carrot brings healty effects for human body. The cooking of the ground parts, especialy leaf, of carrot is very difficult that boiling, frying, frizziling and other cooking method are not able to alternate its tasts.

Futhermore, chewings of the ground parts of carrot is very hard. Therefore, young people, especialy school boys and girls, tend to dislike not only the root but also the leaf of carrot by the above reason.

Fresh juice and fresh vegitable salad making of the raw material of carrot have a defect that is difficult to eat a lot of amounts.

Because, these foods have nonwell smells based on the leaf of carrot. There is other problem on carrot leaf that is not able to clean perfectly for its complexed shape.

In this specification, the word "green-yellow vegitable" means a vegitable having a carotin more than 60 micrgrams per 100 grams in raw materials.

The amounts of carotin is followings in each green-yellow vegitable; carrot 7300 microgram, spinach 3100, pumpkin 790, Spanish paprika 270.

A carrot have more carotin than other green-yellow vegitable.

At the same time, the leaf of the carrot includs a big amount of carotin as same as the root of it, still more the calcium amounts is 5 times than the roots' amounts.

Then, the leaf of the carrot is usually costed away in the farm, because of its easy becoming dirty in calutivation circumstance.

In recent days, circumstance of foods being aculatly and sharply changing on the basis of alternation of food materials and cooking methods brings senility and adult diseases to young or middle age people.

Main reason of this phenomenen is relation to lipids amounts and peroxide lipids amounts in body, and these amounts are relation to increasing of these peoples' high gluose-contain-foods.

-4-

Keeping a good health condition is to be based on the depression of the peroxide lipids amounts in the body, especialy serum.

Thus, the peroxide lipids in serum are causing substance for stopping the blood fluid and destroying the cell wall.

By the way, the population of women having liver spots on skin is progressively increasing and the age of these women is lower and lower than former.

The reasons of the above phenomenen are to be said followings;
1. overeatings of frozen foods including animal lipids and protides,
2. lessereatings of vegitable foods,
3. smokings,
4. keeping late hours, and so on.

The most parts of liver spots origin is unknown, but clearly increasing of peroxide lipids amounts in serum and surface skin (epidelmis.) lipids appears with liver spots.

Citing the Report paper to Association of Japanese Skin Science on 1971 of Dr.Hayakawa of Nagoya University, peroxide lipids is increasing at the time of appearing the skin diseases, i.e. melanoderma and liver spots on face, like as next table A and B.

Table  A

Relations between Skin state and Serum Lipids (persons) .

| Sympton | Total Lipids | Total cholesterols | Peroxid Lipids |
|---|---|---|---|
| Lady's melano-derma on face (8 persons) | 1.23 | 1.66 | 3.6 |
| Liver Spots (7 persons) | 1.09 | 1.19 | 3.5 |
| Dermatitis medicamoutosa (5 persons) | 1.36 | 1.66 | 2.6 |
| Eczema on face (5 persons) | 1.22 | 1.38 | 2.4 |
| Acne | 1.09 | 1.28 | 2.4 |

＊ A verage datum as normal skin being 1.

Table  B

Relations between Skin state and Skin Lipids (persons) .

| Sympton | Total Lipids | Total cholesterols | Peroxid Lipids |
|---|---|---|---|
| Lady's melano-derma on face (8 persons) | 0.7 | 0.7 | 18.8 |
| Liver Spots (7 persons) | 0.5 | 0.4 | 5.3 |
| Dermatitis medicamoutosa (5 persons) | 1.1 | 1.1 | 3.2 |
| Acne | 3.3 | 3.3 | 3.5 |

＊ A verage datum as normal skin being 1.

As aforemention, when the peroxide lipids in serum and epidelmis is increased on the reason of an unblanced diet(overeatings of a froozen animal proteid foods), oxeygen lackings in the serum by smokings, lowering of the cellactivation level by keeping late hours and so on, liver spots the interference in visual acuity or the interference in hair is easily appeared.

Nevertheless the above circumstance, there is nothing of foods, medicines or toilets goods for improvement of various interferences from between internal and external body.

Detail description of the Invention

In this invention, a carrot means 2 years-plants of the Umbellifera family, and this plants' scholar name is Daucus carota L.
Usually, the root parts of this plants, carrot(Daucus carota L.), is eaten. But, only the ground parts of a carrot is used in this invention.

When the ground parts of a carrot is preparedas the component of this inventional anti-oxidation composition, it is necessary to obtain dry powder or extracts powder through drying or extracts methods describing hereinafter.

In this invention, the extract methods are not restriction within following examples.

At first, the leaf parts of a carrot is to be dried with frozen dried methods or natural drying methods.

The crushings of this dried-leaf of a carrot is also used a component of this invention.

The drying process including heating more than 100°C is not preferably use in this invention.
The dried-carrot leaf is extracted with aqueous solvent, if necessary, under reflux.

Futhermore, it is permitted to heat in the above extract process, if it is needed.

The word "aqueous solvents" in this specification means that solvents contains water more than 95 weight %

Preferable examples of aqueous solvents are water, physiological saltwater, alcohol solution, organic acid solution and salt of organic acid solution.

In this extracts process of this invention, both dried and wetted-carrot leaf are able to be employment.

In the case utilizing wetted-carrot, it is necessary to cut the leaf in pieces for pre-treatment.
After this,5 times amounts of water is added on the pre-treatment leaf.

During 2 hours or more, this extraction is proceeded under heating less than 100℃.

After this extraction, the extract solution and solids are filtrated. Filtrated extracts solution is left in silent condition during 24 hours or more under cooling(5 ℃) .

This left extract solution is filtrated again and the filtrated extract solution is condensed to 1/3 times.
This condensed extracts is used intact for the necessary component of this inventional anti-oxidation composition.

Furthermore, drying powder of this condensed extract solution is permitted to use a component of this anti-oxidation composition by means of usual drying method, such as frozen drying.

In the above mentioned example, the other codensed extracts solution or condensed extracts drying powder is obtained to substitute physiological saltwater for water, if necessary.

Organic acid dilute solution, alcohol dilute solution or salt of organic acid dilute solution is able to substitute for water as same operation as the above physiological salt water for obtaining extract powder or condensed extract.

Organic acid including its salts is selected from all organic acids permitted by foods Acts, i.e. acetic acid, citric acid, malic acid or the else.

The concentration of organic acid dilute solution is from 0.1 to 1 weight %, preferably 0.5 weight %.

Tea, i.e. foods style, as the embodiment of this invention's anti-oxidation composition is one of the suitable examples.

It is employed the usual process for making tea from the ground parts or carrot like as Japanese green tea.

One embodiment of making process of the ground parts of carrot (Daucus carota L.) is following approximate procedures;

1). harvesting the ground parts of carrot,
2). cutting the ground parts of carrot in pieces,
3). heating with steam during 10 seconds or more about 98 ℃,
4). rough crumpling during more than 40 minutes under heating at 70 to 75 ℃,

5). fine crumpling during more than 30 minutes under heating at 50 to 55℃,

6). at last, drying during 20 to 30 minutes under heating at 60 to 70 ℃.

Purification of the above extracts with aqueous solvents is able to be acceptance in this invention.

The approximate procedures of purification of the dried-extracts-powder are followings;

1). dissolving the dried-extract-powder in water,

2). filtration this solution,

3). Funnel separating the filtrate solution with n-hexan-water solvents,

4). Taking out the water soluble division,

5). drying this division by water evaporation under diminished pressure,

6). dissolving the dried-division solution into water again,

7). Funnel separating the solution with n-butanol-water,

8). Taking out the water soluble division,

9). drying this division by evaporating the water under diminished pressure,

10).dissolving the dried-division in methanol,

11).Separating the solution with column chromatography means,

12).Finally getting a white powder.

Regretfully the chemical structure of this purified-white-powder is unknown up to this date.

This is a important subject for inventers in near feature.

The healty foods for eyes is the other preferably embodiment of this invention s' anti-oxidation composition.

For deriving more effects from this healty foods for eyes, it is important to mix drying powder or extracts of onion(Allium cepa) and/or citrus fruits (except its juice) with the ground parts of carrot as the necessary component.

The word "onion" means eatable plants belonging the liliacease family and this plant's scholar name is Allium cepa.
In this invention, many variety cultivational species of onion is able to be use, for example, Kaizuka-wase, Aichi-ki-wase, Imai-ki-wase, Awagi-Kodaka, Sapporo-Ki, Yamaguchimaru, Kikai and so on.

Dry powder, especialy frozen dry powder, of onion employs prefrable component of this invention.

The reason of frozen dry powder being preferable than others is to make onion to powder without deviation of onion smell substance, i.e. propyl-allyl-disulfide, allyl-sulfide.

The inventors thought that this onion smell substance assisted the activity of Vitamin Bl being a coenzyme of glucose metabolism in the body.
And the more, this onion smell substance has the function of preventing the fatigue stuff such as lactic acid gathering around the Neuron.

The function of the ground parts of carrot being depression the products of peroxide is more greater by the onion powder.

The peroxide lipids gathering and producting around the eyes especially capillary of eyes is effectively reduced by the above the ground parts of carrot and others component, wherefore the healty effects for eyes is obtained by this anti-oxidation composition.

The mixing ratio between the ground parts of carrot and others is 100, the former, vs. from 50 to 120, the latter, by weight.

In this invention, foods for curing cataract and ingredient of tobacco are other suitable embodiments of this anti-oxidation composition.

It is preferable to mix the dry powder and/or extracts of ginseng(Panax schinseng Ness) with the ground parts of carrot component for the above embodiments.

The word "ginseng" means a plant belonging the Araliaeeae family and this plant scholar name is Panax schinseng Nees. Ginseng is said to be said giving a superior effect for healty and the native land of this ginseng is North-East area of china, korean peninsula and so on.

All parts of this, ginseng including roots and leaf, preferably roots, are permitted to use the material of this invention.

Dried-powder, preferably frozen dried-powder, and/or extracts by the same procedure as the ground parts of carrot is preferably used in this invention. In the case of making this tobacco composition, these components are added in any one process of usual tobacco making procedures that consists of tobacco leaf mixing, processing and molding.

The mixing step is selected by the relation between components state, powder or extract solution, and tobacco making process. The mixing proportion of these components is less than 50 weight % and more than 2 weight % in all tobacco composition.

In the case less than 2 weight % mixing, than effect of this invention is not appeared, on the contrary, in the case more than 50 weight % mixing, the taste and the smell as the tobacco is inferior than no mixing tobacco.

The mixing ratio between the ground part of carrot and ginseng is the former 100 vs. the latter from 50 to 120 by weight.

Now, we describe Examples, Tests and comparisons hereinafter, for effects of this invention being clear.

Example 1

2 kg of leaf parts of ordinaly carrot is dried.

This dried leaf is extracted with 10 ℓ of water during 60 minutes under heating at 80 ℃.

This extraction repeats 3 times.

Then the extract solution was filtrated and condensed 1/10 times of original amounts.

This condensed extracts is frozen dried into powder.

Finaly, 760 grams of dried powder is obtained.

Example 1-1

This test is performed by using the above dried powder named anti-oxidation agent 1.

4 test oils is prepared.

Anti-oxidation agent 1; linoleic acid 10g (wakojunyaku Coltd.,), Zein 80 (Atlas Coltd., surface active agent) 0.8g and the above anti-oxidation agent 0.8g.

Comparison1-1 ;linoleic acid 10g, Zein 80 0.8g and BHA(wakojunyaku Coltd.,) 0.1g.

Comparison1-2; linoleic acid 10g, Zein 80 0.8g and BHT(wakojunyaku Coltd.,)0.1g.

Comparison1-3; linoleic acid 10g, Zein 80 0.8g and α-tchophero(Tokyo Kasei Coltd .,)10.1g

These test oils are put into thermal-control cisterm at 60°C, and aerated for 3 hours by air poump.

The results of depression rate of oxidation after 3 hours are described in table 1-1.

The datum of depression rate are measured by Per Oxide Value and Thio-Balbital Acid Valu(

Example 1-2

The following composition of toilet water is prepared.

example A

| glycerine | 5.0 (%) |
|---|---|
| polyethylene glychol | 0.2 |
| surface active agent | 2.0 |
| oleic alchol | 0.1 |
| ethyl alcohol | 15.0 |
| fragrance | 0.1 |
| anti-oxidant agent 1 | 0.05 |
| purified water | the residual |
| total | 100 ml |

comparison

the same composition only without anti-oxidation agent 1.

Test 1-1

20 persons who are women having a skin eruption from 18 to 45 years are divided into 2 groups.

Each groups' persons uses the above toilet water at morning and night during 2 months, and are investigated at every 2 weeks.

0173181

-14-

The results of this test are described in from table 1-2 to table 1-4.

Test 1-2
3 groups, 10 heads of one group, of male Wister rats are reared.
Group 1 rats are taken usual feed including a purified cornoil at the rate of 10g per body weight 1kg at 3 times in a day.

Group 2 are taken feed including peroxide cornoil at the same rate and same times as the above group 1.

Group 3 rats are taken feed including peroxid corn oil and anti-oxidation agent 1 mixed the rate of 500mg / body weight 1 kg amounts, rate and times of feed are same as group 1.

After 6 weeks rearing, weight is measured and analyzed peroxide lipids of the serum.
The results of these are described in Table 1-5 and Table 1-6.

Example 2-1
Test solution A is prepared the following procedure;
Cutting a usual carrot leaf in piecies, drying this leaf by frozen dry method, adding the 10 times water during 5 hours at 50°C; filtrating this extract, leaving this filtrated solution during 24 hours at 5°C, filtrating this solution again, condensing the filtrated solution into 1/2 times of amounts under deminished pressure at 40 to 50 °C.

Test solution B is prepared the same above procedure except substitution 95 weight % ethyl alcohol for water.
Test solution C is prepared the same above procedure except substitution 30 weight % ethyl alcohol for water.

Test solution D is prepared the same above procedure except substitution 3 weight % ethyl alcohol for water.

$\alpha$-tocophlol is used for comparison.

In test tube, diameter 25mm and length 200mm, linoleic acid 5ml and test solution or comparison solution are mixing, and aerating this mixed solution by 500ml/min air during 2 hours at 60℃.

The PerOxide Value of linoleic acid of starting state and final atate are measured.

The results are described in table 2-1.

Test case 2

Soft drinks are tested like as example 2-1.

Soft drinks' composition:

| | |
|---|---|
| condensed solution of | 3.0(%) |
| example 1,2,3 or comparison. | |
| honey | 0.4 |
| citric acid | 0.2 |
| molasses | 15.0 |
| flavor | 0.3 |
| water | the residual |
| Total | 100 ml |

Solution is obtained like as Test case 2-1.

each example are different from extracts solvents such as example 1 being water, example 2 being physiologocal saltwater, example 3 being 0.5% acetic acid Comparison is tocophelel.

The results of this test are described in table 2-2.

Test case 2-3

Packed tea is made of mixing 0.3g of the extracts powder of the leaf parts of carrot obtained at Example 2-1 and 2g of Japanese tea.

6 men from 45 to 67 years who feels urine-residual, noctoria and no morning erection drink this packed tea as one cup of hot tea twice a day for 2 weeks.

Other 6 men of comparison who feels same complains drink usual Japanese tea like as the above test case.

The results of this test are described in Table 2-3.

Test case 2-4

Soft drinks are prepared like as the above Test case 2-2 except substitution dry powder of carrot leaf obtained at test case 2-2 for condensed extracts.

5 pupils who are pseudomyopia drink this soft drink divided 100ml into 2 times, morning and night for one month.

The result are described in Table 2-4.

Details of Table 2-4

(a) case: 2 pupils rise up 0.3 to 0.8 in right eye and left eye at the degree of visual acuity table index.

The other pupil rise up 0.5 to 0.7in right eye and 0.2 to 0.3 in left eye.

(b) case: one pupil rises up 0.5 to 0.7 in right eye and 0.5 to 0.5 in left eye.

The other pupil rises up 0.3 to 0.6 in right eye and 0.6 to 0.8 in left eye.

Example 3

Dry pewder obtained at example 1 is employed the following composition of foods for care and growing hair.

Composition:

Dried-powder of extract of carrot leaf     350mg
Dried-powder of Yuzu(Citrus junos Sieb.ex Tanaka)
Fruits(except juice)                        600mg
malasses                                      50mg
                        Total              1000mg

Foods of the above  composition is molded in granule .

Test case 3-1.

3 groups are consisted of group 1 being 10 men 32 to 55 years old who complain loss of hair, group 2 being 10 women 30 to 45 years old who complain having a little wrinkness and group 3 being 10 men and women 30 to 55 years old who complain cricking in the back.

Every person is taken the foods obtained at example 3 at 3 times in a day for 6 weeks.

Effects of this intakes are described in Table 3-1.

The proportion of effect in all testers is about 60%.

The test of falling off hair is performed of brushing the hair by dried towel after shampoo and recognization atached piece of hair on towel.

In this test, all of conditions such as shampoo times, shampoo method, shampoo agent and brushing times by dry rowel are regurated.

The tests of wrinkness and criking are performed by answering of testers' feelings.

Test case 3-2

15 old age persons from 55 to 77 years old are taken granulated foods obtained at example 3 at 3 times in a day for 8 weeks.

The degree of readability of newspapers' letter is measured
The test is performed of answering the degree of readability of news paper at just after getting up time.

The result of 2 months after are described in Table 3-2.
The rate of effect is about 67%.

Example 4

Tonic for care and growing hair is prepared using a dry powder obtained at Example 1.
The composition of Tonics :

|  |  |
|---|---|
| Lonolin derivatives | 2 (%) |
| Ehtyl alcohol | 75 (%) |
| Fragrance | 1 |
| Purified water | 21 |
| Dry powder of carrot leaf | 1 |

Test case 4-1

30 men who complain loss of hair takes the above tonics after cleaning a hair every day, brushing a shampooed hair by dried towel, stimulating the head skin by brush.

The results of test after 3 weeks are described in Table 4-1.
The test of falling-out hair is performed like as Test case 3-1.
Effect of hair for care and growing are mesured by the answer of testers' feelings and at the same time the third persons' recognization.

0173181

Test case 4-2

60 men from 34 to 55 years old consisting of persons being loss of hair (group 1 ), persons being partially baldness (group 2) and persons being perfectly baldness (group 3) take the above tonic like as test case 4-1 for 6 months.

The results are gathered by answer of persons at 3 months after and 6 months after.

The results of this test are described in Table 4-2 and 4-3.

Example 5

The following compositions' cosmetics of peel-off is prepared

Composition:

| | |
|---|---|
| dry powder of carrot leaf obtained at example 1 | 10.0(%) |
| extracts of carrot leaf obtained at example 2s' test solution. | 3.5 |
| Zinic dioxide | 18.0 |
| Talc | 20.0 |
| Olive oil | 1.8 |
| Surface active agents(poly oxiethylene solbitan mono lauric acde esther) | 1.0 |
| Glicerine | 7.0 |
| Acetic acid | 0.2 |
| Caoline | 0.1 |
| Total | 100.0 |

Test case 5-1

7 women from 29 to 44 years old having a liver spots take the above cosmetics of peel-off.

At the using time, the above cosmetics is mixed with water, toilets water or milky lotion at the rate of 1:1, after that the mixed cosmetics is painted on the face and dried cosmetics coats are washed off by mild hot water after drying. this treatment is performed after washing face at night.

The result of after 3 months and after 6 months are described in Table 5-3.
In the table, Good effect means more than 2 steps improvement, and Effect means one step improvement.
Aggravation and side-effect are nothing in use this cosmetics.
(Judgement)
(a) Color being darken and border being clear.
(b) Color being un-darken and border being clear.
(c) Color being a little remainder  and border being unclear.
(d) Color and border being unclear.
The average of peroxide value is reduced from 6.8 to 2.8 as the normal skin being 1.
The remarkable improvement case is from 4.8 to 1.5 and the lesser improvement case is from 5.5 to 4.7.

Example 5-2
50ml Soft drinks is prepared by mixing a condesed extract carrot leaf by water and Apple fruits juice.
Apple juice is prepared just before drinking for preventing a oxidation.
Test case 5-2
5 women 32 to 46 age having a clear liver spots on face and no caring take the one bottle of the above soft drinks at morning for 3 months.

The results of one month after and 3 months after are described in Table 5-4.

Example 5-3 and Test Case 5-3

Dry powder of carrot leaf obtained at Example 5-1 is prepared to granulate as usual mehtods like as Jintan (Registered Trade Mark).

5 women from 26 to 51 old years who having clear liver spots and no caring now take one pack ( 2 g) of the above granule foods at 3 times in a day after every diet for 2 months.

The results of Test of after one month and 2 months are described in Table 5-5.

Example 6

The foods of following composition is prepared by mixing dry powder of carrot leaf as obtained Example 1 and powder of onion produced by frozen dry method from usual onion.

1.7 Kg onion powder is made from 2 Kg usual onion.

Composition:

| | |
|---|---|
| Onion powder | 450mg |
| Carrot leaf powder | 50mg |
| Fructose | 225mg |
| Corn starch | 1275mg |
| Total | 2000mg |

Test Case 6-1

20 persons from 25 to 35 years old who continuously operate the Computer and Word Processer for 5 hours in a day take this foods 3 times in a day for 3 weeks.

Effects of this test is measured by answer from tester if the fatigue of eyes is disappeared or not.

The results of this test are described in Table 6-2.

Fuethermore   the other visual acuity trainings is proceeded with this Test.

Example 7-1

The foods of following composition is prepared.

Composition:

Powdered carrot leaf obtained Example 1        64 (%)
Ginseng extracts powder (Ishu sangyo CoLtd.,)  16
Citrus fruits (except juice)                    20

One pack 2 g is prepared in granule.

Example 7-2

20 persons from 18 to 28 years old who are glycosuriac cataract take 2 packs of Example 7-1 at 2 times in a day.

The other 20 persons who are the same as above take 2 packs of Example 7-2 foods at 2  times a day.  The results are in Table 7.

Example 8-1 and 8-2

Component A is prepared like as Example 1.

Component B is prepared by mixing the carrot leaf powder ( Example 1) and ginseng extracts powder in the rate of 1 vs.0.5.

These component A and B are blended into usual cigarrets in the rate of 10 weight %. Cigarret including component is example 8-2 and component B is Example 8-1.

Test case 8-1 and Test case 8-2

30 persons from 18 to 58 years old divide into 3 groups.

Group 8-1 smoks Example 8-1 10 Cigarrets a day, group 8-2 smoks 10 cigarrets Ex.8-2 and Group 8-3 smoks 10 usual cigarets.

The results of this test after 15 days are described in Table 8-1-1 to Table 8-1-7.

Test Case 8-3

The depression rate and 50% depression rate are measured and the results are described in Table 8-2 and 8-3. Comparoison 1 is B.H.A ,Comparison 2 is B.H.A and Comparison 3 is $\alpha$-tocopherol.Test goes like as Example 1-1.

-23-

Table 1-1

|  | TBAV( %) | POV(%) |
|---|---|---|
| Anti-Oxidation agent (1) | 1 0 0 | 1 0 0 |
| Comparison 1-(1) | 1 0 0 | 1 0 0 |
| Comparison 1-(2) | 1 0 0 | 1 0 0 |
| Comparison 1-(3) | 1 7 | 2 0 |

Table 1-2 (After 2 weeks) (Persons)

|  | Good effect | Non-effect |
|---|---|---|
| Example A | 6 | 4 |
| Comparison | 2 | 8 |

Table 1-3 (After 4 weeks) (Persons)

|  | Good effect | Non-effect |
|---|---|---|
| Example A | 7 | 3 |
| Comparison | 3 | 7 |

Table 1-4 (After 6 weeks) (Persons)

|  | Good effect | Non-effect |
|---|---|---|
| Example A | 8 | 2 |
| Comparison | 2 | 8 |

Table 1-5 Deta of Serum Analysis (mg/dl Average)

|  | TC | HDL-ch | TG |
|---|---|---|---|
| Group 1 | 67.3 | 40.7 | 61.6 |
| Group 2 | 172.7 | 32.8 | 378.8 |
| Group 3 | 124.0 | 39.7 | 117.9 |

Table 1-6   Deta of Serum Analysis (Average)

|  | Index of arteriosclerosis | FFA (meq/ml) | LPO (nmol/ml) |
|---|---|---|---|
| Group 1 | 0.37 | 0.15 | 3.03 |
| Group 2 | 0.85 | 1.19 | 10.1 |
| Group 3 | 0.67 | 0.83 | 4.55 |

Table 2-1

| Test solution | Depression rate of POV |
|---|---|
| (A) | 94.0 (%) |
| (B) | 19.3 |
| (C) | 26.5 |
| (D) | 84.3 |
| Comparison | 23.1 |

Table 2-2

| Test liquid | Depression rate of POV |
|---|---|
| Example 1 | 23.0 (%) |
| Example 2 | 24.1 |
| Example 3 | 25.1 |
| Comparison | 23.0 |

Table 2-3   (Persons)

|  | Test case | Comparison case |
|---|---|---|
| No-residual urine feeling | 3 | 1 |
| No-nocturia | 5 | 0 |
| Feelings of morning erection | 6 | 0 |

-25-

Table  2-4

| Improvement degree of visual acuity | Persons |
|---|---|
| (a)Remarkable effect | 3 |
| (b)Remarkable effect | 2 |

Table  3-1 (Persons)

|  | Group  1 | Group  2 | Group  3 |
|---|---|---|---|
| Unchanged | 4 | 5 | 3 |
| Remarkable effect | 2 | 2 | 2 |
| Good effect | 6 | 3 | 5 |

Table  3-2

|  | Persons |
|---|---|
| Unchanged | 5 |
| No-dimness | 7 |
| A little dimness | 3 |

Table  4-1 (Persons)

|  | Group  1 | Group  2 | Group  3 |
|---|---|---|---|
| Unchanged | 4 | 5 | 3 |
| Remarkable effect | 2 | 2 | 2 |
| Good effect | 6 | 3 | 5 |

Table  4-2 (After 3 months) (Persons)

|  | Good effect | Effect | Ineffect | Side effect |
|---|---|---|---|---|
| Group 1 | 4 | 1 | 5 | 0 |
| Group 2 | 4 | 1 | 5 | 0 |
| Group 3 | 0 | 2 | 8 | 0 |

Table 4-3 (After 6 months) (Persons)

|  | Good effect | Effect | Ineffect | Side effect |
|---|---|---|---|---|
| Group 1 | 6 | 2 | 2 | 0 |
| Group 2 | 7 | 1 | 2 | 0 |
| Group 3 | 0 | 3 | 7 | 0 |

Table 5-3 (Persons)

|  | After 3 months | After 6 months |
|---|---|---|
| Good effect | 2 | 4 |
| Effect | 3 | 2 |
| Ineffect | 2 | 1 |

Table 5-4 (Persons)

|  | After 1 months | After 3 months |
|---|---|---|
| Good effect | 1 | 2 |
| Effect | 2 | 3 |
| Ineffect | 2 | 0 |

Table 5-5 (Persons)

|  | After 1 months | After 3 months |
|---|---|---|
| Good effect | 1 | 2 |
| Effect | 3 | 3 |
| Ineffect | 1 | 0 |

Table 6-1 (Persons)

|  | Example |
|---|---|
| Ineffect | 8 |
| Effect | 2 |
| Non-tired | 1 0 |

Table 6-2

|  | Persons |
|---|---|
| Ineffect | 5 |
| Part recovery | 7 |
| Perfect recovery | 3 |

Table 7 (Persons)

|  | Example 7-1 | Example 7-2 |
|---|---|---|
| Unchanged | 6 | 4 |
| Good effect | 2 | 2 |
| Effect | 1 2 | 1 4 |

Table 8-1-1 (Persons)

|  | Test group 8-1 | Test group 8-2 | Test group 8-3 |
|---|---|---|---|
| I.Languor feering | Persons who feel languor. | | |
| Disappearauce | 4 | 2 | 0 |
| Partial-improvement | 3 | 4 | 0 |
| Unchanged | 3 | 4 | 8 |
| Aggrevation | 0 | 0 | 2 |

Table 8-1-2 (Persons)

|  | Test group 8-1 | Test group 8-2 | Test group 8-3 |
|---|---|---|---|
| II.Bitter feelings in the oral cavity | Persons who chronical feels bitter at the time of rising. | | |
| Disappearauce | 5 | 2 | 0 |
| Partial-improvement | 2 | 4 | 0 |
| Unchanged | 3 | 4 | 8 |
| Aggrevation | 0 | 0 | 2 |

0173181

Table   8-1-3 (Persons)

| | Test group 8-1 | Test group 8-2 | Test group 8-3 |
|---|---|---|---|
| III.Discomfort of stomach | Persons who chronical feels discomfort of stomach. | | |
| Disappearance | 2 | 2 | 0 |
| Partial-improvement | 4 | 7 | 0 |
| Unchanged | 4 | 1 | 6 |
| Aggrevation | 0 | 0 | 4 |

Table   8-1-4(Persons)

| | Test group 8-1 | Test group 8-2 | Test group 8-3 |
|---|---|---|---|
| IV.Roughen skin | Persons who feels no good coordination toilet goods with overdrying skin. | | |
| Good effect | 9 | 7 | 0 |
| Un changed | 1 | 3 | 10 |

Table   8-1-5(Persons)

| | Test group 8-1 | Test group 8-2 | Test group 8-3 |
|---|---|---|---|
| V.Abnormal evacuation | Persons who is in the condition of repeated obstipation or diarrhea. | | |
| Recovery | 3 | 2 | 0 |
| Almost recovery | 4 | 2 | 0 |
| Unchanged | 3 | 6 | 10 |

Table    8-1-6(Persons)

|  | Test group 8-1 | Test group 8-2 | Test group 8-3 |
|---|---|---|---|
| VI.Palpitation | Persons who feels palpitation at the time of running up the upstairs. | | |
| Improvement | 2 | 2 | 0 |
| Almost recovery | 4 | 5 | 1 |
| Unchanged | 4 | 3 | 9 |

Table    8-1-7 (Persons)

|  | Test group 8-1 | Test group 8-2 | Test group 8-3 |
|---|---|---|---|
| VII.Sleeplessness | Persons who often awaken in nights. | | |
| Sleep soundly | 3 | 3 | 0 |
| Reducing the times of awakingness in nights | 5 | 3 | 0 |
| Unchanged | 2 | 4 | 10 |

Table    8-2 (Persons)

|  | TBAV ( %) | POV (%) |
|---|---|---|
| Component A | 100 | 100 |
| Component B | 100 | 100 |
| Comparison 1 | 100 | 100 |
| Comparison 2 | 100 | 100 |
| Comparison 3 | 20 | 17 |

-30-

Table 8-2

|              | TBAV( 50%)          | POV(50%)            |
| ------------ | ------------------- | ------------------- |
| Component A  | $2.02 \times 0.01$  | $1.93 \times 0.01$  |
| Component B  | $1.25 \times 0.01$  | $1.21 \times 0.01$  |
| Conparison 1 | $3.37 \times 0.001$ | $3.75 \times 0.001$ |
| Conparison 2 | $1.92 \times 0.001$ | $2.24 \times 0.001$ |
| Conparison 3 | $1.95 \times 0.1$   | $2.48 \times 0$     |

CLAIMS:

1. Anti-oxidation composition which consists of the ground parts of carrot (Daucus carota L.) as a necessary component.

2. Anti-oxidation composition as set forth in claim 1, which is characterized in that the ground parts of carrot (Daucus carota L.) is dry powder and /or extracts.

3. Anti-oxidation composition as set forth in claim 1 and 2, which is characterized in that the said extacts is extracted with aqueous solvents.

4. Anti-oxidation composition as set forth in claim 1,2 and 3, which is characterized in that the said aqueous solvents is physiological saltwater.

5. Anti-oxidation composition as set forth in claim 1,2 and 3, which is characterized in that the said aqueous solvents is alcohol dilute solution.

6. Anti-oxidation composition as set forth in claim 1,2 and 3, which is characterized in that the said aqueous solvents is organic acid dilute solution.

7. Anti-oxidation composition as set forth in claim 1,2 and 3, which is characterized in that the said aqueous solvents is salt of organic acid dilute solution.

8. Anti-oxidation composition as set forth in claim 1,2,3,4,5,6 and 7, which is characterized in that the said dry powder and/or extracts is treated in heating process less than 100°C.

9. Anti-oxidation composition which consists of the ground parts of carrot (Daucus carota L.), and at least one's dry powder and/or extracts selected from the group consisting of onion (Allium cepa), ginseng (Panax schinseng Nees) and citrus fruits (except its juice).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 101, no. 3, 16th July 1984, page 494, no. 22158p, Columbus, Ohio, US; W. BOCK et al.: "Antioxidative action of native lignin from carrots", NAHRUNG 1984, 28(3), 273-8 * Abstract * | 1 | C 09 K 15/34<br>A 23 L 3/34 |
| | --- | | |
| X | FOOD SCIENCE AND TECHNOLOGY ABSTRACTS, 84005872 (84-01-t0017); L.A. FOMICHEVA et al.: "Activity of vegetable additives used for food concentrates", & MOSKOVSKII TEKH. INST. PISHCHEVOI PROMYSHLENNOSTI, MOSCOW, USSR, KONSERVNAYA I OVOSHCHESUSHIL'NAYA PROMYSHLENNOST', no. 1, 39-41, 1982 * Abstract * | 1,8,9 | |
| | ---      -/- | | **TECHNICAL FIELDS SEARCHED (Int. Cl.4)**<br><br>C 09 K |

The present search report has been drawn up for all claims

| Place of search THE HAGUE | Date of completion of the search 27-11-1985 | Examiner BOULON A.F.J. |
|---|---|---|

European Patent
Office

**EUROPEAN SEARCH REPORT**

0173181
Application number

EP 85 11 0262

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FOOD SCIENCE AND TECHNOLOGY ABSTRACTS, 84065768 (84-11-g0810); V.N. GULYAEV et al.: "Extending the storage time of food concentrates", & NAUCHNO-ISSLED. INST. PISHCHEKONTSENTRATNOI PROMYSHLENNOSTI I SPETSIAL'NOI PISHCHEVOI TEKH., USSR, KONSERVNAYA I OVOSHCHESUSHIL'NAYA PROMYSHLENNOST', no. 9, 25, 1983 * Abstract * | 1 | |
| X | FOOD SCIENCE AND TECHNOLOGY ABSTRACTS, 85012592 (85-02-t0005); L.A. FOMITSCH et al.: "Effect of preserving foods using additives of plant origin. Die Konservierungswirkung von pflanzlichen Zusatzen in Lebensmitteln", & MOSCOW TECH. INST. OF THE FOOD IND., MOSCOW, USSR ERNAHRUNG, vol. 8, no. 2, p.81-83, 1 ref. 1984 * Abstract * | 1 | **TECHNICAL FIELDS SEARCHED (Int. Cl.4)** |

--- -/-

The present search report has been drawn up for all claims

| Place of search THE HAGUE | Date of completion of the search 27-11-1985 | Examiner BOULON A.F.J. |
|---|---|---|

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 70. no. 3, 20th January 1969, page 166, no. 10502z, Columbus, Ohio, US; A. IVANOVA et al.: "Oxidative changes in sunflower oil during heat treatment with some types of vegetables", & ISV. INST. KHRANENE, BULG. AKAD. NAUK. 1968, 7, 93-9 * Abstract * | 1 | |

TECHNICAL FIELDS SEARCHED (Int. Cl.4)

The present search report has been drawn up for all claims

| Place of search | Date of completion of the search | Examiner |
|---|---|---|
| THE HAGUE | 27-11-1985 | BOULON A.F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503. 03.82